(19) Europäisches Patentamt
European Patent Office
Office européen des brevets

(11) Veröffentlichungsnummer: **0 012 932**
**B1**

# (12) EUROPÄISCHE PATENTSCHRIFT

(45) Veröffentlichungstag der Patentschrift: **20.01.82**

(51) Int. Cl.[3]: **C 07 D 239/30**

(21) Anmeldenummer: **79105101.4**

(22) Anmeldetag: **12.12.79**

## (54) Verfahren zur Herstellung von 2,4,5-Trichlorpyrimidin.

(30) Priorität: **22.12.78 DE 2855660**

(43) Veröffentlichungstag der Anmeldung: **09.07.80 Patentblatt 80/14**

(45) Bekanntmachung des Hinweises auf die Patenterteilung: **20.01.82 Patentblatt 82/3**

(84) Benannte Vertragsstaaten: **BE CH DE FR GB IT**

(56) Entgegenhaltungen:
**ANGEWANDTE CHEMIE, 82. Jahrgang 1970. Nr. 2 Weinheim DE R. Braden et al (Bayer) «Direktsynthese chlorierter Pyrimidine», Seiten 78, 79**

(73) Patentinhaber: **BAYER AG, Zentralbereich Patente, Marken und Lizenzen, D-5090 Leverkusen 1, Bayerwerk (DE)**

(72) Erfinder: **Döring, Fritz, Am Hang 13, D-5068 Odenthal (DE)**
Erfinder: **Beck, Gunther, Dr., Am Mittelberg 19, D-5090 Leverkusen (DE)**
Erfinder: **Dankert, Gerhard, Dr., Arthur-Hantzsch-Strasse 44, D-5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG.

# Verfahren zur Herstellung von 2,4,5-Trichlorpyrimidin

Gegenstand der vorliegenden Erfindung ist ein neues Verfahren zur Herstellung von 2,4,5-Trichlorpyrimidin.

Das Verfahren ist dadurch gekennzeichnet, dass man (2-Cyanethyl)-isocyaniddichlorid der Formel

$$NC-CH_2-CH_2-N=CCl_2 \quad (I)$$

vorzugsweise in einem inerten Lösungsmittel – zunächst mit Chlorwasserstoff und anschliessend mit Chlor umsetzt.

Zum Stand der Technik ist folgendes zu bemerken.

1) Nach dem Verfahren der DE-OS 2 701 797 (Anmeldetag: 18.1.77, Offenlegungstag: 20.7.78) werden zum Erhalt von 2,4,5-Trichlorpyrimidin Verbindungen der Formel

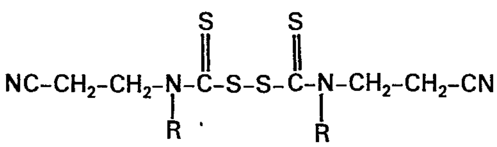

worin R für einen unter den Reaktionsbedingungen abspaltbaren Rest steht, bei Temperaturen von 0–40 °C mit mehr als 7 und weniger als 13 Mol Chlor, insbesondere 11 Mol Chlor umgesetzt und anschliessend wird in Abwesenheit von Chlor auf Temperaturen von etwa 100–150 °C, insbesondere 110–140 °C, nacherhitzt.

2) Nach dem Verfahren der DE-OS 2 725 888 (Anmeldetag: 8.6.77, Offenlegungstag: 21.12.78) werden zum Erhalt von 2,4,5-Trichlorpyrimidin Verbindungen der Formel

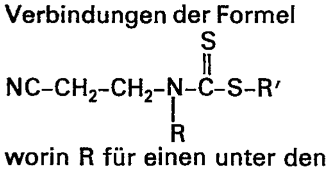

worin R für einen unter den Reaktionsbedingungen abspaltbarer Rest und

R′ für einen gegebenenfalls substituierten niederen Alkylrest steht, bei Temperaturen von 0 bis 50 °C mit weniger als 7,5 Mol Chlor, insbesondere 3,5 bis 7,0 Mol Chlor umgesetzt und anschliessend wird in Abwesenheit von Chlor auf Temperaturen von 100–150 °C, insbesondere 110–140 °C, nacherhitzt.

Beide Verfahren zeigen in bezug auf das erfindungsgemässe Verfahren zur Herstellung von 2,4,5-Trichlorpyrimidin folgende entscheidende Nachteile:

Als zwangsläufige Koppelprodukte entstehen bei beiden Umsetzungen 1) und 2) grosse Mengen störender Schwefelchloride.

Weiterhin entsteht aus dem jeweiligen Rest R der allgemeinen Formeln in 1) und 2) durch Abspaltung hochchlorierter Verbindungen (insbesondere chlorierte Kohlenwasserstoffe) ein unerwünschtes Nebenprodukt, welches wegen der Gefahr der Umweltbelastung als chlorierter Kohlenwasserstoff aufwendig technisch abgetrennt bzw. beseitigt werden muss.

Die Umsetzung mit Chlorwasserstoff erfolgt beim erfindungsgemässen Verfahren im allgemeinen im Temperaturbereich von –20 bis 50 °C, vorzugsweise zwischen 0 und 30 °C. Dabei bildet sich ein farbloser Niederschlag. Man leitet im allgemeinen so lange Chlorwasserstoff ein, bis sich kein weiterer Niederschlag mehr bildet. Im allgemeinen benötigt man bis zur vollständig beendeten Niederschlagsbildung 2 Mole Chlorwasserstoffe pro Mol (I). Höhere Mengen an Chlorwasserstoff sind für die Reaktion nicht nachteilig.

Die Reaktion kann auch bei erhöhtem HCl-Druck durchgeführt werden. Naturgemäss ist die Geschwindigkeit der Reaktion mit Chlorwasserstoff auch druckabhängig, d.h. schnellere Reaktion bei höherem HCl-Druck.

Bei CHl-Normaldruck erfolgt im allgemeinen weitgehend vollständige Umsetzung in etwa 5–30 Stunden, vorzugsweise 10–20 Stunden, unter erhöhtem Druck entsprechend kürzer.

Die Umsetzung mit Chlorwasserstoff ist jedoch auch abhängig von der Art des verwendeten inerten Lösungsmittels. Als inerte Lösungsmittel seien genannt:

2,4,5-Trichlorpyrimidin selbst, Phosphoroxychlorid, Thionylchlorid, Chloroform.

Besonders bevorzugt sind 2,4,5-Trichlorpyrimidin und Phosphoroxychlorid. In diesen Lösungsmitteln erfolgt bei HCl-Normaldruck im Bereich von 10–20 Stunden praktisch vollständige Umsetzung, während in Chloroform und in Thionylchlorid in vergleichbaren Zeiträumen die Umsetzung mit Chlorwasserstoff noch nicht vollständig ist. In diesen Fällen kann jedoch bei der destillativen Aufarbeitung der Chlorierungsansätze nicht umgesetztes (2-Cyanethyl)-isocyaniddichlorid durch fraktionierte Destillation vom gebildeten 2,4,5-Trichlorpyrimidin abgetrennt und einer erneuten Umsetzung mit HCl und Chlor zugeführt werden.

Die im Anschluss an die Umsetzung mit Chlorwasserstoff folgende Umsetzung mit Chlor wird im allgemeinen bei etwa 40–150 °C, vorzugsweise bei etwa 50–80 °C durchgeführt. Man leitet dabei so lange Chlor ein, bis keine weitere Aufnahme mehr erfolgt, mindestens aber so lange, bis der durch die vorhergehende Umsetzung mit Chlorwasserstoff gebildete Niederschlag vollständig in die Lösung gegangen ist. Dazu benötigt man – vorausgesetzt, dass die Umsetzung mit HCl quantitativ war – im allgemeinen etwa 2 Mole Chlor pro Mol (I). Man kann selbstverständlich mit einem Überschuss an Chlor arbeiten.

Das Ende der Umsetzung mit Chlor erkennt man im allgemeinen daran, dass die Suspension in eine homogene Lösung übergegangen ist. Diese Hauptchlorierungsreaktion wird vorzugsweise zwischen 50 und 80 °C durchgeführt. In der Praxis chloriert man zwecks Erhöhung der Ausbeute an 2,4,5-Trichlorpyrimidin auch nach dem Erreichen der homogenen Lösung noch etwa 2–5 Stunden im leicht überschüssigen Chlorstrom (erkennbar

an der grünlichen Farbe des Abgases) weiter. Für diese Nachchlorierungsreaktion genügt die obere Grenze des bevorzugten Temperaturbereiches der Hauptchlorierungsreaktion, also z.B. 80°C; man kann aber auch während der Nachchlorierungsreaktion die Temperatur bis auf 150°C steigern.

Zur Vermeidung einer geringfügigen Weiterchlorierung des gebildeten bzw. des als Lösungsmittel eingesetzten 2,4,5-Trichlorpyrimidins zu Tetrachlorpyrimidin ist es zweckmässig, die gesamte Chlorierungsreaktion unter Lichtausschluss durchzuführen.

Zur Durchführung des erfindungsgemässen Verfahrens mischt man die flüssige Ausgangsverbindung der Formel (I) mit 2–20, vorzugsweise 5–10 Gewichtsteilen eines der genannten inerten Lösungsmittel und leitet HCl-Gas (mindestens 2 Mol pro Mol I) im Verlauf von 5–30, vorzugsweise 10–20 Stunden ein. Man kann aber auch die gesamte, zum Einsatz kommende Menge HCl gleich zu Begin in dem Gemisch aus (I) und Lösungsmittel auflösen und danach bis zur beendeten Umsetzung weiterrühren. Die anschliessende Chlorierung erfolgt dann vorzugsweise bei 50–80°C, gegebenenfalls bis 150°C, zweckmässig unter Lichtausschluss.

Die Umsetzungen mit HCl und/oder mit Chlor können selbstverständlich auch kontinuierlich gestaltet werden.

Die Aufarbeitung erfolgt in üblicher Weise durch fraktionierte Destillation.

2,4,5-Trichlorpyrimidin kann durch Gasphasenchlorierung (GB-PS 1 201 228) in Tetrachlorpyrimidin umgewandelt werden. Tetrachlorpyrimidin ist als Reaktivkomponente für die Herstellung von Reaktivfarbstoffen geeignet (vgl. z.B. Belgische Patentschrift Nr. 578 933). Darüber hinaus besitzt 2,4,5-Trichlorpyrimidin fungizide und sporizide Eigenschaften (vgl. US-Patentschrift Nr. 3 227 612) und lässt sich für die Herstellung von Reaktivfarbstoffen mit Dichlorpyrimidylgruppen verwenden, wobei als Ausgangsverbindungen Farbstoffe mit Amino- oder Amidgruppen eingesetzt werden.

Die Herstellung von (2-Cyanethyl)-isocyaniddichlorid (I) erfolgt beispielsweise durch Umsetzung von (2-Cyanethyl)-formamid (II) mit Thionylchlorid und Chlor gemäss der Reaktionsgleichung

$NC-CH_2-CH_2-NH-CHO + SOCl_2 + Cl_2 \longrightarrow$

(II)

$NC-CH_2-CH_2-N=CCl_2 + SO_2 + 2HCl$

(I)

bei Temperaturen von 40–80°C, vorzugsweise 50–75°C. Anstelle von Thionylchlorid kann man dabei vorteilhafterweise auch entsprechende Mengen Phosgen gemäss der Reaktionsgleichung

$NC-CH_2-CH_2-NH-CHO + COCl_2 + Cl_2 \longrightarrow$

(II)

$NC-CH_2-CH_2-N=CCl_2 + CO_2 + 2HCl$

(I)

einsetzen.

(2-Cyanethyl)-formamid (II) kann in bekannter Weise (Französische Patentschrift 976 959) z.B. aus Formamid und Acrylnitril hergestellt werden.

Die Herstellung von (I) mittels Phosgen und Chlor erfolgt in inerten Lösungsmitteln wie z.B. Chloroform oder 2,4,5-Trichlorpyrimidin. Dies ermöglicht die Herstellung von 2,4,5-Trichlorpyrimidin aus (2-Cyanethyl)-formamid (II) ohne Zwischenisolierung von (2-Cyanethyl)-isocyaniddichlorid (I) im «Eintopf»-Verfahren.

Gegenstand der vorliegenden Erfindung ist demzufolge weiterhin ein Verfahren zur Herstellung von 2,4,5-Trichlorpyrimidin, das dadurch gekennzeichnet ist, dass man zunächst (2-Cyanethyl)-formamid (II) mit einem Gemisch aus Phosgen und Chlor in einem inerten Lösungsmittel bei Temperaturen von 40–80°C, vorzugsweise 45–70°C zu (2-Cyanethyl)-isocyaniddichlorid (I) umsetzt und dieses anschliessend gegebenenfalls ohne Zwischenisolierung zunächst mit Chlorwasserstoff bei −20 bis 50°C, vorzugsweise 0–30°C, und dann mit Chlor bei 40–150°C, vorzugsweise 50–80°C umsetzt – wie oben beschrieben.

Beispiel 1

In eine Mischung aus 930 g Phosphoroxychlorid und 151 g (1,0 Mol) (2-Cyanethyl)-isocyaniddichlorid wurden bei etwa 5°C im Verlauf von 2 Stunden 100 g (2,74 Mol) Chlorwasserstoff eingeleitet und anschliessend 20 Stunden im gleichen Temperaturbereich nachgerührt. In die so entstandene Suspension wurde nach Aufheizen auf 60°C im Temperaturbereich von 60–70°C innerhalb 1,5 Stunden 280 g (3,94 Mol) Chlor eingeleitet, wobei der Niederschlag vollständig in Lösung ging. Anschliessend wurden bei 80°C innerhalb 5 Stunden weitere 100 g (1,4 Mol) Chlor durch die Lösung geleitet. Während der gesamten Chlorierung wurde ohne Lichtzufuhr gearbeitet. Die Aufarbeitung des Reaktionssatzes erfolgte durch fraktionierte Destillation im Wasserstrahlvakuum. Nach dem Abziehen des Phosphoroxychlorids bis zu einem Siedepunkt von 70°C bei 133 mbar erhielt man bei Kp (16 mbar) 90–108°C 196 g Destillat, das nach der gaschromatographischen Analyse neben 7,5% Phosphoroxychlorid 91,4% (entsprechend 179 g) 2,4,5-Trichlorpyrimidin enthielt; das entspricht einer Ausbeute von 97,5% der Theorie.

Ein praktisch reines Produkt lässt sich durch Rektifikation an einer Kolonne von etwa 1 m Länge bei Kp (16 mbar) 94–96°C erhalten.

Beispiel 2

In eine Mischung aus 1150 g 2,4,5-Trichlorpyri-

midin und 198 g (1,31 Mol) (2-Cyanethyl)-isocyaniddichlorid wurden bei etwa 25 °C im Verlauf von 15 Stunden 150 g (4,11 Mol) Chlorwasserstoff eingeleitet. In die dicke Suspension wurden sodann bei 60–80 °C unter Lichtausschluss innerhalb 3 Stunden 240 g (3,38 Mol) Chlor eingeleitet, wobei sich eine klare, hellgelbe Lösung bildete. Innerhalb weiterer 3 Stunden wurde die Innentemperatur bis auf 150 °C gesteigert, wobei stündlich 20 g Chlor eingeleitet wurden. Die anschliessende Destillation im Wasserstrahlvakuum lieferte bei Kp (17 mbar) 95–110 °C 1435 g Destillat, das nach der gaschromatographischen Analyse zu 96,3% (entsprechend 1380 g) aus 2,4,5-Trichlorpyrimidin bestand. Das entspricht einer Ausbeute von 230 g (95,5% der Theorie) 2,4,5-Trichlorpyrimidin.

Beispiel 3

In eine Mischung aus 1 l reinem Chloroform und 200 g (1,325 Mol) 2-Cyanethyl)-isocyaniddichlorid wurden bei etwa 20 °C im Verlauf von 15 Stunden 150 g (4,11 Mol) Chlorwasserstoff eingeleitet. Anschliessend wurde die Suspension auf 60 °C erwärmt und im Bereich von 10 Stunden unter Lichtausschluss insgesamt 300 g Chlor eingeleitet, worauf eine klare Lösung entstand. Unter weiterem Einleiten von 20 g Chlor/Stunde wurde die Temperatur im Verlauf von 5 Stunden auf 150 °C gebracht, wobei gleichzeitig über eine 30 cm lange Kolonne Chloroform abdestillierte. Anschliessend wurde im Wasserstrahlvakuum destilliert. Man erhielt bei Kp (16 mbar) 90–110 °C 178 g Destillat, das nach der gaschromatographischen Analyse zu 61,0% (entsprechend 108,5 g) aus 2,4,5-Trichlorpyrimidin und zu 38,2% (entsprechend 68 g) aus unumgesetztem (2-Cyanethyl)-isocyaniddichlorid bestand. Das entspricht einer Ausbeute von 67,6% 2,4,5-Trichlorpyrimidin, bezogen auf umgesetztes (2-Cyanethyl)-isocyaniddichlorid.

Beispiel 4

In eine Mischung aus 800 ml Thionylchlorid und 200 g (1,325 Mol) (2-Cyanethyl)-isocyaniddichlorid wurden bei 20–25 °C im Verlauf von 12 Stunden 120 g (3,29 Mol) Chlorwasserstoff eingeleitet. In die gut rührbare Suspension wurden sodann bei 50 °C unter Lichtausschluss innerhalb 3 Stunden 240 g (3,38 Mol) Chlor eingeleitet, wodurch eine klare Lösung entstand. Unter weiterem Einleiten von stündlich ca. 20 g Chlor wurde die Innentemperatur im Verlauf von 5 Stunden auf 150 °C gesteigert, wobei gleichzeitig über eine Kolonne Thionylchlorid abdestillierte. Die anschliessende destillative Aufarbeitung lieferte bei Kp (17 mbar) 95–110 °C 172 g Destillat, das nach der gaschromatographischen Analyse zu 84,3% (entsprechend 145 g) aus 2,4,5-Trichlorpyrimidin und zu 11,7% (entsprechend 21 g) aus unumgesetztem (2-Cyanethyl)-isocyaniddichlorid bestand. Das entspricht einer Ausbeute von 66,7% 2,4,5-Trichlorpyrimidin, bezogen auf umgesetztes (2-Cyanethyl)-isocyaniddichlorid.

Beispiel 5

Herstellung von 2,4,5-Trichlorpyrimidin aus (2-Cyanethyl)-formamid ohne Zwischenisolierung von (2-Cyanethyl)-isocyaniddichlorid («Eintopf»-Verfahren):

In 980 g 2,4,5-Trichlorpyrimidin wurden bei 45 °C 100 g (1,01 Mol) Phosgen gelöst und anschliessend bei gleicher Temperatur im Verlauf von 2 Stunden 130 g (1,325 Mol) (2-Cyanethyl)-formamid mittels Dosierpumpe sowie 158 g (2,22 Mol) Chlor und 150 g (1,51 Mol) Phosgen über Strömungsmesser eindosiert. Nach etwa 30minütigem Nachrühren bei 45–50 °C war die Gasentwicklung beendet. Anschliessend wurde auf 90 °C geheizt und durch Anlegen eines Vakuums von ca. 100 Torr überschüssiges Phosgen abgezogen.

Nach dem Abkühlen auf 25 °C wurden im Verlauf von 15 Stunden 150 g (4,11 Mol) Chlorwasserstoff eingeleitet. Anschliessend erfolgte bei 60–65 °C unter Lichtausschluss die Chlorierung, die unter Einleiten von 200 g (2,82 Mol) Chlor im Zeitraum von 2 Stunden die zu Beginn kräftige Suspension in eine homogene Lösung überführte. Unter Einleiten von 20 g Chlor pro Stunde wurde die Temperatur innerhalb 4 Stunden auf 150 °C gesteigert. Danach wurde destillativ aufgearbeitet. Man erhielt bei Kp (16 mbar) 92–108 °C 1195 g Destillat, das nach der gaschromatographischen Analyse zu 97,9% (entsprechend 1170 g) aus 2,4,5-Trichlorpyrimidin bestand. Das entspricht einer Ausbeute von 190 g (78,2% der Theorie) 2,4,5-Trichlorpyrimidin.

Herstellung des Ausgangsproduktes (2-Cyanethyl)-isocyaniddichlorid:

a) mit Thionylchlorid/Chlor

In einer 4-Liter-Rührapparatur wurden 2200 g (18,48 Mol) Thionylchlorid vorgelegt und bei 55 °C im Verlauf von 4 Stunden gleichzeitig 453 g (4,62 Mol) (2-Cyanethyl)-formamid zugetropft und 365 g (5,14 Mol) Chlor eingeleitet. Nach etwa 30minütigem Nachrühren bei 55–60 °C war die Gasentwicklung weitgehend beendet. Die Aufarbeitung erfolgte durch Destillation. Man erhielt bei Kp (16 mbar) 100–110 °C 663 g (2-Cyanethyl)-isocyaniddichlorid in einer gaschromatographisch ermittelten Reinheit von 95% (entsprechend 95% der Theorie).

b) mit Phosgen/Chlor

In 780 g reines, bei 45 °C mit Phosgen gesättigtes Chloroform wurden bei 45 °C im Verlauf von 2 Stunden gleichzeitig 130 g (1,325 Mol) (2-Cyanethyl)-formamid zugetropft und 115 g (1,67 Mol) Chlor sowie 105 g (1,06 Mol) Phosgen eingeleitet. Nach einstündigem Nachrühren bei 50 °C unter weiterem Einleiten von etwa 60 g Chlor wurde destillativ aufgearbeitet. Nach dem Abziehen des Chloroforms erhielt man bei Kp (26 mbar) 110–120 °C 180 g (2-Cyanethyl)-isocyaniddichlorid in einer gaschromatographisch ermittelten Reinheit von 99,7% (entsprechend 90% der Theorie).

Die Herstellung von (2-Cyanethyl)-isocyaniddichlorid nach b) kann in einer geeigneten, aus 3

Rührkolben bestehenden Reaktionskaskade mit praktisch gleicher Ausbeute kontinuierlich gestaltet werden.

Beispiel 6

In eine Mischung aus 1000 g Trichlorpyrimidin und 151 g (1,0 Mol) (2-Cyanethyl)-isocyaniddichlorid wurden im Verlauf von 3 Stunden bei 10 °C 110 g (3,0 Mol) Chlorwasserstoff gelöst und anschliessend 12 Stunden nachgerührt. Nach dem Aufheizen der dabei entstandenen Suspension auf 80 °C wurde mit etwa 50 g/Stunde Chlor im Verlauf von 8 Stunden unter Lichtausschluss chloriert, wobei eine klare Lösung entstand. Anschliessend wurde in die Lösung weitere 4 Stunden lang 20 g/Stunde Chlor bei 80 °C eingeleitet. Die destillative Aufarbeitung lieferte bei Kp (16 mbar) 94–103 °C 1169 g Destillat, das nach der gaschromatographischen Analyse zu 98,2% (entsprechend 1148 g) aus 2,4,5-Trichlorpyrimidin und zu 1,5% (entsprechend 17 g) aus unumgesetztem (2-Cyanethyl)-isocyaniddichlorid bestand. Ausbeute an 2,4,5-Trichlorpyrimidin 90,9%, bezogen auf unumgesetztes (2-Cyanethyl)-isocyaniddichlorid.

**Patentansprüche**

1. Verfahren zur Herstellung von 2,4,5-Trichlorpyrimidin, dadurch gekennzeichnet, dass man 2-Cyanethylisocyaniddichlorid mit Chlorwasserstoff und anschliessend mit Chlor umsetzt.

2. Verfahren nach Anspruch 1, dadurch gekennzeichnet, dass die Umsetzung mit Chlorwasserstoff bei -20 °C bis +50 °C, vorzugsweise zwischen 0 und 30 °C erfolgt.

3. Verfahren nach Ansprüchen 1 und 2, dadurch gekennzeichnet, dass man mit etwa 2 Mol Chlorwasserstoff pro Mol (2-Cyanethyl)-isocyaniddichlorid umsetzt.

4. Verfahren nach Ansprüchen 1–3, dadurch gekennzeichnet, dass man bei 40–150 °C, vorzugsweise bei 50–80 °C mit Chlor umsetzt.

5. Verfahren nach Ansprüchen 1–4, dadurch gekennzeichnet, dass man mit etwa 2 Mol Chlor pro Mol (2-Cyanethyl)-isocyaniddichlorid umsetzt.

6. Verfahren nach Ansprüchen 1–5, dadurch gekennzeichnet, dass man in Gegenwart eines inerten Lösungsmittels, vorzugsweise 2,4,5-Trichlorpyrimidin oder Phosphoroxychlorid arbeitet.

7. Verfahren zur Herstellung von 2,4,5-Trichlorpyrimidin nach Anspruch 1, dadurch gekennzeichnet, dass man zunächst (2-Cyanethyl)-formamid mit einem Gemisch aus Phosgen und Chlor in einem inerten Lösungsmittel bei Temperaturen von 40–80 °C, vorzugsweise 45–70 °C zu (2-Cyanethyl)-isocyaniddichlorid umsetzt und dieses anschliessend ohne Zwischenisolierung zunächst mit Chlorwasserstoff bei -20 bis 50 °C, vorzugsweise 0–30 °C, und dann mit Chlor bei 40–150 °C, vorzugsweise 50–80 °C umsetzt.

**Claims**

1. Process for the preparation of 2,4,5-trichloropyrimidine, characterised in that 2-cyanoethyl isocyanide dichloride is reacted with hydrogen chloride and the product is then reacted with chlorine.

2. Process according to Claim 1, characterised in that the reaction with hydrogen chloride is carried out at -20 °C to +50 °C, preferably between 0 and 30 °C.

3. Process according to Claims 1 and 2, characterised in that the reaction is carried out with about 2 mols of hydrogen chloride per mol of 2-cyanoethyl isocyanide dichloride.

4. Process according to Claims 1-3, characterised in that the reaction with chlorine is carried out at 40-150 °C, preferably at 50-80 °C.

5. Process according to Claims 1-4, characterised in that the reaction is carried out with about 2 mols of chlorine per mol of 2-cyanoethyl isocyanide dichloride.

6. Process according to Claims 1-5, characterised in that the reaction is carried out in the presence of an inert solvent, preferably 2,4,5-trichloropyrimidine or phosphorus oxychloride.

7. Process for the preparation of 2,4,5-trichloropyrimidine according to Claim 1, characterised in that 2-cyanoethyl formamide is first reacted with a mixture of phosgene and chlorine in an inert solvent at temperatures of 40–80 °C, preferably 45–70 °C to give 2-cyanoethyl isocyanide dichloride and this is then first reacted without intermediate isolation with hydrogen chloride at -20 to 50 °C, preferably 0–30 °C and the product is then reacted with chlorine at 40–150 °C, preferably 50–80 °C.

**Revendications**

1. Procédé de préparation de 2,4,5-trichloropyrimidine, caractérisé en ce qu'on fait réagir du dichlorure de (2-cyanéthyl)-isocyanure avec du chlorure d'hydrogène, puis avec du chlore.

2. Procédé suivant la revendication 1, caractérisé en ce que la réaction avec le chlorure d'hydrogène a lieu à une température comprise entre -20°C et +50°C, de préférence, entre 0 et 30°C.

3. Procédé suivant les revendications 1 et 2, caractérisé en ce qu'on effectue la réaction avec environ 2 moles de chlorure d'hydrogène par mole de dichlorure de (2-cyanéthyl)-isocyanure.

4. Procédé suivant les revendications 1 à 3, caractérisé en ce qu'on effectue la réaction avec le chlore à une température de 40 à 150 °C, de préférence, de 50 à 80 °C.

5. Procédé suivant les revendications 1 à 4, caractérisé en ce qu'on effectue la réaction avec environ 2 moles de chlore par mole de dichlorure de (2-cyanéthyl)-isocyanure.

6. Procédé suivant les revendications 1 à 5, caractérisé en ce qu'on travaille en présence d'un solvant inerte, de préférence, la 2,4,5-trichloropyrimidine ou l'oxychlorure de phosphore.

7. Procédé de préparation de 2,4,5-trichloropyrimidine suivant la revendication 1, caractérisé en ce qu'on fait tout d'abord réagir du (2-cyanéthyl)-formamide avec un mélange de phosgène

et de chlore dans un solvant inerte à des températures de 40 à 80°C, de préférence, de 45 à 70°C pour obtenir le dichlorure de (2-cyanéthyl)-isocyanure que l'on fait ensuite réagir, sans isolation intermédiaire, tout d'abord avec du chlorure d'hydrogène à une température comprise entre −20 et 50°C, de préférence, entre 0 et 30°C, puis avec du chlore à une température comprise entre 40 et 150°C, de préférence, entre 50 et 80°C.